# EUROPEAN PATENT SPECIFICATION

(11) **EP 3 444 336 B1**
(45) Date of publication and mention of the grant of the patent: **08.05.2024**
(21) Application number: 18189272.0
(22) Date of filing: 16.08.2018
(51) Int. Cl.: C12N 9/02, C11D 3/386

(54) **CLEANING AGENT**
REINIGUNGSMITTEL
AGENT DE NETTOYAGE

(30) Priority: 18.08.2017 EP 17186883
(43) Date of publication of application: 20.02.2019
(73) Proprietor: The Procter & Gamble Company, Cincinnati, OH 45202 (US)
(72) Inventor: LANT, Neil Joseph, Newcastle upon Tyne, NE12 9TS (GB); Patterson, Steven George, Newcastle upon Tyne, NE12 9TS (GB); Vasquez Valdivieso, Montserrat Guadalupe, Newcastle upon Tyne, NE12 9TS (GB)
(74) Representative: P&G Patent Belgium UK

(56) References cited:
- EP-B1- 2 350 250
- WO-A1-2007/036235
- WO-A1-2007/112679
- WO-A1-2014/067933
- US-A1- 2016 312 157

## Description

### TECHNICAL FIELD

The present invention is in the field of cleaning. It relates to a cleaning agent, in particular, a cleaning agent comprising a supporting substrate with an oxidoreductase-enzyme immobilized thereon. The invention also relates to a cleaning kit comprising the cleaning agent and a method of cleaning using the cleaning agent. The invention provides better cleaning by decolourising the wash liquor, preventing and/or reducing soil re-deposition and malodour while caring for the surface cleaned.

### BACKGROUND OF THE INVENTION

When cleaning a surface by immersion in a wash liquor, dirt goes from the surface to be cleaned to the liquor. Dirt encompasses stains, soils, malodours, bacteria, etc. Dirt can be redeposited onto the surface being cleaned. There can also be transfer of colour from the surface being cleaned to the liquor. Colour bleeding can occur during the cleaning of a surface with a wash liquor. During the cleaning process dyes can migrate from the surface to the wash liquor. These dyes can be deposited onto other surfaces immersed in the wash liquor impairing on the appearance of the surface, similarly colours coming from stains can also be deposited onto the surface being cleaned. This can be more apparent in the case of laundry loads containing white fabrics. The white fabrics tend to become greyish when washed in the presence of fabrics that are not completely white. Dyes in the wash liquor can also contribute to colour deterioration of coloured fabrics. Soils, stains, bacteria, malodours removed from the fabrics can also being re-deposited on the fabrics in detriment of the cleaning process.

In the case of mixed laundry loads, i.e. loads containing coloured and white fabrics, or in the case of multi-colours fabrics bleach should not be used because it could alter the colours of coloured fabrics. This can also be the case when cleaning patterned hard-surfaces.

Cleaning products being sold in the market can comprise enzymes. The enzymes usually found in laundry detergents are amylase, cellulase, protease and/or lipase. These enzymes are used in detergents as cleaning and fabric care agents. In order to remove stains, these enzymes typically first need to deposit onto the stains. Amylase, cellulase, protease and lipase have been immobilized on various substrates. For example, US 2013/0316430 A1 describes the immobilization of amylase, cellulase, protease and lipase on a PVC surface, in particular on to a plastic bucket and a brush for their application in cloth washing. WO 2014/006424 A1 is directed to a cleaning formulation comprising a multiplicity of solid cleaning particles, wherein said solid cleaning particles comprise polymeric particles and at least one cleaning agent, wherein said at least one cleaning agent is immobilised on the surface of said polymeric particles. Enzymes are among the cleaning agents recited in `424. In the case of `430 and `424 cleaning of fabrics seems to work by slowly releasing the enzymes into the wash liquor to access the fabrics.

WO2015/185393 A1 relates to a detergent containing at least one oxidoreductase as a colour transfer inhibitor however; the oxidoreductase would attack the dyes on the fabrics. EP 2 350 250 B1 describes an enzyme/substrate co-delivery system. The liquid delivery system includes at least one enzyme encapsulated in a water-soluble polymeric matrix and a substrate for the enzyme in a carrier liquid in which the polymeric matrix is insoluble. WO 2007/036235 A1 describes the immobilization of enzymes by adsorbing enzymes, a polyfunctional amine and a cross-linking agent onto a particulate porous carrier in a mixer apparatus or in a fluid bed apparatus. US 2016/0312157 A1 describes a detergent pouch, formed by an enzymaticwater-soluble film, comprising a detergent where the water-soluble film contains enzyme particles. WO 2007/112679 A1 describes silk fibroin nanoparticles fixed with enzyme and their production method. WO 2014/067933 A1 describes the use of insoluble enzyme preparations comprising an enzyme which is immobilized on a solid carrier having an average particle size of from about 0.1 to 500 µm as additives in consumer near applications.

The object of the present invention is to provide improved cleaning and at the same time protect the colour of surfaces. In particular, to provide cleaning while caring for the colours of coloured fabrics and prevent the greying of white fabrics in mixed loads as well as preventing malodours and soil re-deposition. It is also desirable to reduce and/or prevent bacterial growth.

### SUMMARY OF THE INVENTION

According to the first aspect of the invention, there is provided a cleaning agent as defined by claim 1. The invention also provides, as defined by claim 6, a cleaning kit. The invention also provides, as defined by claim 11, a method for cleaning surfaces. The invention also provides, as defined by claim 12, a use of the cleaning agent for cleaning a surface. Further features are defined by the dependent claims. The cleaning agent comprises an oxidoreductase-enzyme supported on a supporting substrate by means of a covalent bond. The oxidoreductase-enzyme is selected from oxidoreductase-enzymes belonging to the group having an enzyme classification number selected from the group consisting of E.C. 1.1 - 1.10 and E.C. 1.12 - 1.99.

A "cleaning agent" within the meaning of the invention is an agent that can be used in a cleaning process to contribute to the cleaning on its own but preferably in combination with a cleaning composition.

A "supporting substrate" within the meaning of the invention is any substrate capable of having an enzyme immobilised on its surface.

A "oxidoreductase-enzyme" is an enzyme that catalyzes the transfer of electrons from one molecule to another. The electron transfer can contribute to the decolourization of dyes and can help to avoid soil re-deposition and malodour and it can additionally prevent bacteria growth in the wash liquor. The object of this invention is to improve cleaning. This is achieved by reducing dyes decolourization and preventing soil re-deposition and malodour on the surfaces being cleaned. This is achieved by immobilizing the oxidoreductase. Thus, the transfer of electrons would take place in the wash liquor, wherein the cleaning agent is located, and not on the surface to be cleaned, this would result in a cleaner wash liquor that would be translated into cleaner surfaces without exposing the surface to the chemical aggression that oxidoreductase enzymes can present.

A "mediator" within the meaning of the invention is a small redox molecule that acts as an electron carrier between the supporting substrate to be oxidised and an oxidoreductase enzyme. Once the mediator is oxidised, by giving one or several of its electrons to the oxidoreductase enzyme, it can diffuse into the wash liquor in the proximity of the oxidoreductase enzyme to oxidise dyes, soils, malodour, bacteria, etc, thereby cleaning the wash liquor and resulting in better cleaning. Mediators interact with the oxidoreductase enzyme to ensure the flow of electrons in the case of strongly different redox potentials between the oxidizing enzymes and substrate upon which the enzyme will act.

Colour bleed can occur when fabrics, or any other surfaces, get wet and dye leaches out of the fibers. This commonly occurs in the washing machine and can result in colour transfer between items in the load.

There are two different ways to attack a dye, chemically, to remove its colour. One is by *oxidation,* in which electrons are removed, while the other is by *reduction,* in which electrons are added.

Chromophores cause colours by reflecting a certain portion of the visible spectrum of light. For example, a blue fabric contains chromophores that reflect blue light that our eyes see as the colour blue.

An oxidizing agent works by breaking the chemical bonds of a chromophore (part of a molecule that has colour). This changes the molecule so that it either has no colour or else reflects colour outside the visible spectrum.

A reducing agent works by changing the double bonds of a chromophore into single bonds. This alters the optical properties of the molecule, making it colourless.

The oxidoreductase enzyme is immobilised on a supporting substrate. The immobilization of the oxidoreductase enzyme makes the oxidation process take place where or in close proximity to where the oxidoreductase enzyme is located rather than on the surface to be cleaned. As discussed before, this results in better cleaning while caring for the cleaned surfaces. This differs from a traditional cleaning process where the enzymatic activity and bleaching take place on the soils deposited on the surfaces to be cleaned.

The oxidoreductase enzyme is preferably immobilised on the substrate by means of a chemical bond. The supporting substrate can be selected from the group consisting of fabrics, non-woven materials, plastics and inorganic particles. In particular, substrates in the form of a tri-dimensional hollow body that favours the flow of wash liquor through, or into and out of, it are preferred herein. Plastic supporting substrates in the form of a tri-dimensional hollow body are preferred for use herein, more preferably when the oxidoreductase enzyme is immobilised on the inside of the hollow body. An additional advantage of the cleaning agent of the invention is that it can be re-used because the oxidoreductase enzyme is not consumed in the cleaning process. In particular it has been found easy to re-use the cleaning agent in the case in which the supporting substrate is a fabric, non-woven material or shaped plastic article such as a tri-dimensional hollow body, such that these supporting substrates are preferred. Also preferred are inorganic particles having a large surface area such as zeolites.

According to a second aspect of the invention, there is provided a cleaning kit comprising a cleaning composition and the cleaning agent of the invention. In some embodiments, the cleaning composition comprises a mediator, preferably selected from the group consisting of organic-based mediator, transition metal coordination complex mediator and mixtures thereof.

According to a third aspect of the invention, there is provided a method for cleaning a surface comprising contacting the (first) surface with a wash liquor in a (first) wash step, the wash liquor comprising a cleaning composition comprising the cleaning agent of the invention. In a preferred aspect of the invention, the cleaning agent of the invention is re-used. Thus, the cleaning agent of the invention is separated from the wash liquor of the first wash step and is re-used in a second wash step in which a second surface is contacted with a (second) wash liquor, the second wash liquor comprising a cleaning composition comprising the cleaning agent of the invention. There is also provided a method of cleaning using the cleaning kit of the invention. The method of the invention is applicable to any type of surfaces, including hard surfaces and soft surfaces. The method of the invention is especially suitable for the cleaning of fabrics, in particular, for the cleaning of fabrics of mixed colours. The method avoids greying of the fabrics. The method of the invention may be particularly preferred to reduce dye transfer in the wash liquor.

The elements of the cleaning agent of the invention described in connection with the first aspect of the invention apply *mutatis mutandis* to the second and third aspects of the invention.

### DETAILED DESCRIPTION OF THE INVENTION

The present invention envisages a cleaning agent comprising a supporting substrate with an oxidoreductase enzyme immobilized on the supporting substrate by means of a covalent bond, a cleaning kit comprising the cleaning agent and a method of cleaning using a cleaning composition and the cleaning agent.

As used herein, articles, for example, "a" and "an" when used in a claim, are understood to mean one or more of what is claimed or described.

### Immobilisation

Immobilisation of the oxidoreductase enzyme on the supporting substrate is achieved by covalent bonds.

Immobilisation of the oxidoreductase enzyme is preferably achieved by treating the supporting substrate with at least one activating agent in order to modify the chemical properties at the surfaces of the substrate in order that the modified substrate may subsequently be treated with at least one oxidoreductase enzyme in order to facilitate immobilization of the oxidoreductase enzyme.

The activated supporting substrate can then be further treated with a linking agent which facilitates attachment of the oxidoreductase enzyme by means of a covalent bond.

Activation of the surface may also be achieved by the use of physical agents, such as heat or electromagnetic radiation, e.g. ultra-violet radiation or microwave radiation prior to reaction with a linking agent.

Suitable linking agents may include glutaraldehyde, or may be selected from, for example, typical crosslinking agents such as dimethyl adipimidate, dimethyl suberimidate, pentafluorophenyl ester, hydroxymethyl phosphine, imidoesters and N-hydroxysuccinimide esters.

Other suitable linking agents include, for example:
N-Hydroxysuccinimide (NHS) and N-(3-Dimethylaminopropyl)-N'-ethylcarbodiimide hydrochloride (EDC);
Acylimidazoles (e.g. Carbonyl Diimidazole (CDI) and N,N'-carbonylbis(3- methylimidazolium) triflate (CBMIT);
Phosphonium salts (e.g. benzotriazol-1-yl-oxy-tris-(dimethylamino)-phosphonium hexafluorophosphate (BOP);
Uronium salts (e.g. 0-((ethoxycarbonyl)cyanomethylene amino)-N,N,N',N'- tetramethyl-uronium tetrafluoroborate (TOTU); and
Mukaiyama's reagent (2-chloro-1-methylpyridinium iodide).

Alternatively, embodiments utilising activating agents may include the treatment of polymeric particles incorporating polar groups, including for example Nylon 6,6 or polyethylene terephthalate), initially with a polar group-containing material - such as, for example, gelatin, starch, cellulose, chitosan, chitan, carboxymethylcellulose, poly(vinylimidazoles), poly(acrylic acid), poly(methacrylic acid), poly(lactic acid), poly(maleic acid), poly(glycolic acid), poly(acrylonitrile), polyvinylpyrrolidone), poly(dimethylaminoethyl methacrylate), poly(ethylene imine), poly(allylamine), poly(allylamine) hydrochloride, poly(ethylene glycol), polypropylene glycol), poly(acrylamide), polyvinyl alcohol), polyvinyl acetate), polyvinyl formamide), poly(vinylamine), amine-containing molecules (including biomolecules such as proteins), carboxylic acids such as maleic acid and itaconic acid, and carboxylic acid-containing polymers, as well as derivatives and copolymers of all the foregoing - wherein ionic interactions are formed between the polymer particles and a layer of the polar group-containing material, and subsequently with the oxidoreductase enzyme wherein further ionic interactions are established between the layer of polar group-containing material and the layer of oxidoreductase enzyme.

Optionally, said embodiments utilising at least one activating agent may comprise multiple treatments with the at least one activating agent and/or multiple subsequent treatments or reactions with the at least one mediator. Said embodiments, which rely on ionic interactions, do not require the use of a linker.

### Substrates

A variety of materials that can be used as supporting substrate for immobilization of the mediator include polymeric materials (plastics), including natural or synthetic or partially synthetic polymeric materials for example, cellulose, polystyrene, gelatin, agar, acrylate polymers such as poly(2-hydroxyethyl methacrylate), poly (methyl methacrylate-acrylic acid), polyacrylamide, acrylonitrile/acrylamide polymers, polyesters, alginates, poly (vinyl alcohol) PVA, polyurethane, homo or copolymers. These may be in any form, for example, the substrate may be in the form of a moulded article, sheet, film, woven or non-woven article, fibres, foam, gel, bead, spheres. Preferred examples include cellulose, polystyrene, alkylamine glass beads through covalent coupling, cation exchange resin, photographic gelatin, plastic supports, agar gel, acrylonitrile/acrylamide membranes, poly(2-hydroxyethyl methacrylate) microspheres, poly (methyl methacrylate-acrylic acid) microspheres, polyacrylamide gel, glass beads, sodium alginate beads, superporous celbeads, polyester surface free and affixed alkyl and arylamine glass beads, alginate gel beads, cyclic carbonate bearing hybrid materials, cellulose fibre materials and cellulose-coated magnetite (CCM) nanoparticles.

Other preferred materials suitable as supporting substrate for immobilization of the mediator include polyurethane foam, tri(4-formyl phenoxy) cyanurate, polyacrylamide-acrylic gel, acrylamide grafted acrylonitrile copolymer (PAN), chemically modified pumic particles, nanofibrous poly (vinyl alcohol) PVA, passive epoxy acrylate films modified by magnetic filtered plasma stream, silicate clay mineral, modified polyvinyl alcohol, coated chitosan beads, loofa sponge, liposomes, brick dust via glutaraldehyde and silicon wafers of amino terminated surface.

Other suitable substrates for immobilization of the mediator are particles, preferably selected from inorganic particles, however, some organic particles can also be used. A preferred supporting substrate herein is selected from the group consisting of a silica particle, a zeolite, an aluminum oxide, an organic polymer having either a carboxyl or an amino group, and a mixture thereof. These organic polymers are, preferably, selected from the group consisting of a polyacrylic acid, a polymaleic acid, a poly peptide, chitosan and a mixture thereof. Preferably, the supporting substrate has a median particle size (as measured as the diameter of the particle) of from about 1 nanometer to about 10 micrometers, more preferably, from about 1 nanometer to about 1 micrometer and even more preferably, the supporting substrate is selected from a silica having a particle size of from about 5 nanometers to about 1 micrometer. The median particle size is measured by SEM (Scanning Electron Microscope). A highly preferred silica is SiOx (MN1P, which is provided by Zhou Shan Ming Ri Nano Material Company (Zhejiang Province, China). Other preferred supporting substrates are described in PCT patent publication No. WO 90/04181 which is assigned to Nilsson, published on Apr. 19, 1990.

When an inorganic particle is selected as the supporting substrate, it can be modified by a linking molecule before being activated. Any compounds which can provide the substrate with either carboxyl and/or amino groups can be used as a linking molecule herein. A suitable linking molecule can be a silane linking molecule, preferably the structure of the silane molecule is R₁-(CH₂)ₙ₁-Si(O(CH₂)ₙ₂CH₃)₃, wherein R₁ is selected from -COOH or -NH₂; n1 is from about 1 to about 16, preferably from about 3 to about 8; n2 is from about 0 to about 10, preferably from about 0 to about 4. A preferred linking molecule for use herein is 3-aminopropyltriethoxysilane (APS). The weight ratio of the linking molecule to the substrate is preferably from about 0.001: 1 to about 10:1, and more preferably from about 0.1: 1 to about 5:1. Other linking molecules useful herein are described in U.S. Pat. No. 6,004,786 to Yamashita, et al., issued Dec. 21, 1999.

The linking molecule modifies the supporting substrate to connect the substrate and the oxidoreductase enzyme. In some instances, it is preferred to add a functional group introducer together with the linking molecule to the supporting substrate. A preferred functional group introducer is a carboxylic group introducer or an amino group introducer, more preferably a carboxylic group introducer such as a carboxylic acid anhydride. It is conceivable that the linking molecule itself may sometimes work as the functional group introducer. For example, when selecting carboxylic silane as the linking molecule, an additional functional group introducer is not necessary.

The modification of the substrate by the linking molecule or functional group introducer can be accomplished by mixing the supporting substrate with the linking molecule with functional group introducer into a common organic solvent such as toluene, and re-fluxing for from about 4 hours to about 7 hours, preferably about 6 hours. The refluxed mixture is extracted by filtration, washed with ethanol and dried at about 30° C to about 70° C, preferably from about 45° C to about 55° C, for 20 minutes. The mixture is preferably kept in the vacuum dry container until being applied to next step.

Preferred carboxylic acid anhydrides are selected from the group consisting of a succinic anhydride, a maleic anhydrides, or a mixture thereof. In order to link a carboxyl group onto the substrate, the substrate is usually dissolved in organic solvents, preferably, a mixture of pyridine and anhydrous diethylether, and is mixed with a carboxylic acid anhydride at 25° C, for 17 hours. After mixing, the mixture is extracted by filtration and washed with organic solvents, preferably, anhydrous diethylether is used.

After the substrate has been modified, an activating molecule activates the substrate to connect the oxidoreductase enzyme onto the substrate. The activation can be performed by adding an activating molecule to the activated substrate and stirring together for from about 30 minutes to about 60 minutes, at 4° C. A preferable activating molecule for use herein is a water soluble carbon diimide. More preferably, the water soluble carbon diimide is selected from the group consisting of ethyl-3-(3-dimethyaminopropyl)-carbon diimide hydrochloride (EDC), a succinimide, and a mixture thereof. The weight ratio of the activating molecule to the substrate is preferably from about 0.01: 1 to about 1:1, more preferably, from about 0.05: 1 to about 0.5:1. After the substrate is activated, the supporting substrate is isolated by centrifuging the sample and decanting the supernatant.

A suitable way to immobilize oxidoreductase enzymes is by coating the substrate with polyphenol. The coating can be formed on diverse material surfaces under mild aqueous conditions. Examples of polyphenols include tannic acid, pyrogallol, pyrogallol 2-aminoethane, dopamine, etc, tannic acid is preferred for use herein.

### Substrate configuration

The substrate can have any configuration but it would preferably have a configuration that promotes the contact between the mediator and the wash liquor and avoid the contact with the surface to be cleaned. Preferably, the substrate will be a tri-dimensional hollow body and the mediator would be placed on the inside of the hollow body. Other preferred substrates for use herein are particles in which the oxidoreductase enzyme has been immobilized in the internal surface of the particle. Zeolites are preferred for use herein. Non-woven substrates are also preferred for use herein.

### Oxidoreductase enzyme

The cleaning composition of the invention preferably comprises oxidoreductase enzyme from the enzyme classification E.C. 1.1 - 1.10 and E.C. 1.12 - 1.99.

Preferred oxidoreductase enzyme for use herein include any laccase enzyme comprised by the enzyme classification (EC 1.10.3.2), any chatechol oxidase enzyme comprised by the enzyme classification (EC 1.10.3.1). Additionally any monophenol monooxygenase enzyme comprised by the enzyme classification (EC 1.14.99.1); any bilirubin oxidase enzyme comprised by the enzyme classification (EC 1.3.3.5). Other commercially available oxidoreductase enzymes include ascorbate oxidase, cellobiose dehydrogenase, glucose oxidase, hexose oxidase and sulfhydryl oxidase.

Preferably the oxidoreductase enzyme of the invention is a laccase. Preferred laccases of the present invention include:
a) variants of the wild-type laccase from *Myceliophthora thermophila* which has at least 70%, preferably at least 80% identity for amino acid sequence SEQ ID NO:1.
b) variants of the wild-type laccase from *Bacillus licheniformis* which has at least 70%, preferably at least 80% identity for amino acid sequence SEQ ID NO:2.
c) variants of the wild-type laccase from *Streptomyces sviceus* which has at least 70%, preferably at least 80% identity for amino acid sequence SEQ ID NO:3.

### Mediator

In some embodiments, the cleaning kit of the invention comprises an oxidoreductase-mediator (a mediator for the oxidoreductase enzyme).

A mediator is a redox molecule (typically small) that acts as an electron carrier between the substrate to be oxidised and the oxidising enzyme. Once the mediator is oxidised, by giving one or several of its electrons to the oxidoreductase enzyme, it oxidises dyes, malodours, bacteria, soil, etc. The oxidoreductase enzyme then gains electrons from the substrate that is oxidised to return to its reduced state, making it available once again for oxidation by the oxidoreductase enzyme. Overall, the oxidoreductase-mediator system acts as a catalyst to oxidise substances in the wash liquor. In the present case, the immobilized mediators are activated by an oxidoreductase enzyme. The mediators according to the invention include the chemical structure: 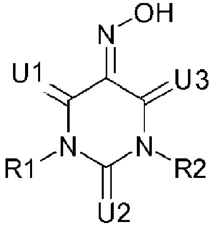 wherein U1 , U2 and U3 are identical or different, and are O, S or NOH; and R1 and R2 are identical or different, and are hydrogen, hydroxyl, formyl, carbamoyl or sulfono radical, ester or salt of the sulfono radical, sulfamoyl, nitro, nitroso, amino, cyano, phenyl, benzyl, CrC4-alkyl, Ci-C4-alkoxy, Ci-C4-carbonyl, carbonyl-Ci-C4-alkyl.

In an embodiment, U1, U2 and U3 are identical or different, and are O or S; and R1 and R2 are identical or different, and are hydrogen, hydroxyl, formyl, carbamoyl or sulfono radical, ester or salt of the sulfono radical, sulfamoyl, nitro, nitroso, amino, cyano, phenyl, benzyl, Ci-C4- alkyl, Ci-C4-alkoxy, Ci-C4-carbonyl, carbonyl-Ci-C4-alkyl.

In another embodiment, U1, U2 and U3 are O; and R1 and R2 are identical or different, and are hydrogen, hydroxyl, formyl, carbamoyl or sulfono radical, ester or salt of the sulfono radical, sulfamoyl, nitro, nitroso, amino, cyano, phenyl, benzyl, Ci-C4-alkyl, Ci-C4-alkoxy, Ci-C4-carbonyl, carbonyl-Ci-C4-alkyl.

In another embodiment, U1, U2 and U3 are identical or different, and are O, S or NOH; and R1 and R2 are identical or different, and are hydrogen, hydroxyl, methyl, ethyl, phenyl, benzyl, formyl, amino, cyano, nitroso, methoxy and/or ethoxy. In another embodiment, U1, U2 and U3 are identical or different, and are O or S; and R1 and R2 are identical or different, and are hydrogen, hydroxyl, methyl, ethyl, phenyl, benzyl, formyl, amino, cyano, nitroso, methoxy and/or ethoxy. In another embodiment, U1, U2 and U3 are O; and R1 and R2 are identical or different, and are hydrogen, hydroxyl, methyl, ethyl, phenyl, benzyl, formyl, amino, cyano, nitroso, methoxy and/or ethoxy.

Mediators could be 1 -methylvioluric acid, 1 ,3-dimethylvioluric acid, thiovioluric acid and violuric acid (alloxan-4,5-dioxime).

The mediator could also be alloxan-5-oxime (violuric acid) and/or its esters, ethers or salts.

Examples of enhancers and mediators are disclosed in EP 705327; WO 98/56899; EP677102; EP 781328; and EP 707637. If desired a distinction could be made by defining an oxidoreductase enzyme system (e.g. a laccase, or a peroxidase enzyme system) as the combination of the enzyme in question and its acceptor, and optionally also an enhancer and/or mediator for the enzyme in question.

Another mediator is hydroxyl benzoate and hydroxyl benzotriazole.

The mediator may be selected from the group consisting of aliphatic, cyclo- aliphatic, heterocyclic or aromatic compounds containing the moiety >N-OH. The mediator could include a compound of the general formula I: 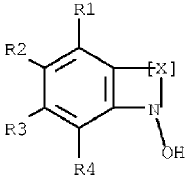 wherein R1 , R2, R3, R4 are individually selected from the group consisting of hydrogen, halogen, hydroxy, formyl, carboxy and salts and esters thereof, amino, nitro, Ci-i2-alkyl, Ci-6-alkoxy, carbonyl(Ci-i2-alkyl), aryl, in particular phenyl, sulfo, aminosulfonyl, carbamoyl, phosphono, phosphonooxy, and salts and esters thereof, wherein the R1 , R2, R3, R4 may be substituted with R5, wherein R5 represents hydrogen, halogen, hydroxy, formyl, carboxy and salts and esters thereof, amino, nitro, Ci-i2-alkyl, Ci-6-alkoxy, carbonyl(Ci-i2-alkyl), aryl, in particular phenyl, sulfo, aminosulfonyl, carbamoyl, phosphono, phosphonooxy, and salts and esters thereof;
[X] represents a group selected from (-N=N-), (-N=CR6-)m, (-CR6=N-)m, (-CR7=CR8-)m, (-CR6=N- NR7-), (-N=N-CHR6-), (-N=CR6-N R7-), (-N=CR6-CH R7-), (-CR6=N-CHR7-), (-CR6=CR7-NR8-), and (-CR6=CR7-CH R8-), wherein R6, R7, and R8 independently of each other are selected from H, OH, NH2, COOH, S03H, Ci-6-alkyl, N02, CN, CI, Br, F, CH2OCH3, OCH3, and COOCH3; and m is 1 or 2.

The term "C1-n-alkyl" wherein n can be from 2 through 12, as used herein, represent a branched or straight alkyl group having from one to the specified number of carbon atoms. Typical Ci-6-alkyl groups include, but are not limited to, methyl, ethyl, n-propyl, iso-propyl, butyl, iso-butyl, sec-butyl, tert-butyl, pentyl, iso-pentyl, hexyl, iso-hexyl and the like.

The mediator could include a compound of the general formula II : 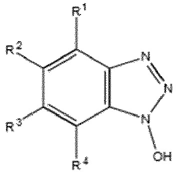 wherein R1, R2, R3, R4 are individually selected from the group consisting of hydrogen, halogen, hydroxy, formyl, carboxy and salts and esters thereof, amino, nitro, Ci-i2-alkyl, Ci-6-alkoxy, carbonyl(Ci-i2-alkyl), aryl, in particular phenyl, sulfo, aminosulfonyl, carbamoyl, phosphono, phosphonooxy, and salts and esters thereof, wherein the R1 , R2, R3, R4 may be substituted with R5, wherein R5 represents hydrogen, halogen, hydroxy, formyl, carboxy and salts and esters thereof, amino, nitro, Ci-i2-alkyl, Ci-6-alkoxy, carbonyl(Ci-i2-alkyl), aryl, in particular phenyl, sulfo, aminosulfonyl, carbamoyl, phosphono, phosphonooxy, and salts and esters thereof.

The mediator may also be a salt or an ester of formula I or II .

The mediator may also be oxoderivatives and N-hydroxy derivatives of heterocyclic compounds and oximes of oxo- and formyl-derivatives of heterocyclic compounds, said heterocyclic compounds including five-membered nitrogen-containing heterocycles, in particular pyrrol, pyrazole and imidazole and their hydrogenated counterparts (e.g. pyrrolidine) as well as triazoles, such as 1 ,2,4-triazole; six-membered nitrogen-containing heterocycles, in particular mono-, di- and triazinanes (such as piperidine and piperazine), morpholine and their unsaturated counterparts (e.g. pyridine and pyrimidine); and condensed heterocycles containing the above heterocycles as substructures, e.g. indole, benzothiazole, quinoline and benzoazepine.

Examples of mediators from these classes of compounds are pyridine aldoximes; N-hydroxypyrrolidinediones such as N-hydroxysuccinimide and N- hydroxyphthalimide; 3,4-dihydro-3-hydroxybenzo[1 ,2,3]triazine-4-one; formaldoxime trimer (N,N',N"-trihydroxy-1 ,3,5-triazinane); and violuric acid (1 ,3-diazinane-2,4,5,6-tetrone-5-oxime).

Other mediators which may be applied in the invention include oximes of oxo- and formyl-derivatives of aromatic compounds, such as benzoquinone dioxime and salicylaldoxime (2-hydroxybenzaldehyde oxime), and N-hydroxyamides and N-hydroxyanilides, such as N-hydroxyacetanilide.

Mediators could also be selected from the group consisting of 1 - hydroxybenzotriazole; 1 - hydroxybenzotriazole hydrate; 1 -hydroxybenzotriazole sodium salt; 1 - hydroxybenzotriazole potassium salt; 1 -hydroxybenzotriazole lithium salt; 1 - hydroxybenzotriazole ammonium salt; 1 -hydroxybenzotriazole calcium salt; 1 - hydroxybenzotriazole magnesium salt; and 1 - hydroxybenzotriazole-6-sulphonic acid.

All the specifications of N-hydroxy compounds above are understood to include tautomeric forms such as N-oxides whenever relevant.

Another group of mediators comprises a -CO-NOH- group and has the general formula III: 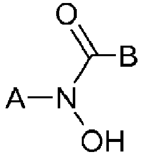 in which A is: 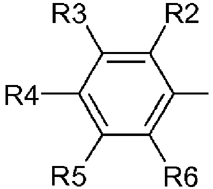 and B is the same as A; or B is H or Ci-i2-alkyl, said alkyl may contain hydroxy, ester or ether groups (e.g. wherein the ether oxygen is directly attached to A-N(OH)C=0-, thus including N-hydroxy carbamic acid ester derivatives), and R2, R3, R4, R5 and R6 independently of each other are H, OH, N H2, COOH, S03H , d-8-alkyl, acyl, N02, CN, CI, Br, F, CF3, NOH-CO-phenyl, CO-NOH-phenyl, Ci-6-CO-NOH-A, CO-NOH-A, COR12, phenyl-CO-NOH-A, OR7, NR8R9, COOR10, or NOH-CO-R1 1 , wherein R7, R8, R9, R10, R1 1 and R12 are C1-12-alkyl or acyl. R2, R3, R4, R5 and R6 of A are preferably H, OH, NH2, COOH, S03H, C1-3-alkyl, acyl, N02, CN, CI, Br, F, CF3, NOH-CO-phenyl, CO-NOH-phenyl, COR12, OR7, NR8R9, COOR10, or NOH-CO-R1 1 , wherein R7, R8 and R9 are d-3-alkyl or acyl, and R10, R1 1 and R12 are Ci-3-alkyl; more preferably R2, R3, R4, R5 and R6 of A are H, OH, NH2, COOH, S03H, CH3, acyl, N02, CN, CI, Br, F, CF3, CO-NOH-phenyl, COCH3, OR7, NR8R9, or COOCH3, wherein R7, R8 and R9 are CH3 or COCH3; even more preferably R2, R3, R4, R5 and R6 of A are H, OH, COOH, S03H, CH3, acyl, N02, CN, CI, Br, F, CO-NOH-phenyl, OCH3, COCH3, or COOCH3; and in particular R2, R3, R4, R5 and R6 of A are H, OH, COOH, S03H, CH3, N02, CN, CI, Br, CO-NOH-phenyl, or OCH3.

R2, R3, R4, R5 and R6 of B are preferably H, OH, NH2, COOH, S03H, C1-3-alkyl, acyl, N02, CN, CI, Br, F, CF3, NOH-CO-phenyl, CO-NOH-phenyl, COR12, OR7, NR8R9, COOR10, or NOH-CO-R1 1 , wherein R7, R8 and R9 are C1-3-alkyl or acyl, and R10, R1 1 and R12 are Ci-3-alkyi; more preferably R2, R3, R4, R5 and R6 of B are H, OH, NH2, COOH, S03H, CH3, acyl, N02, CN, CI, Br, F, CF3, CO-NOH-phenyl, COCH3, OR7, NR8R9, or COOCH3, wherein R7, R8 and R9 are CH3 or COCH3; even more preferably R2, R3, R4, R5 and R6 of B are H, OH, COOH, S03H, CH3, acyl, N02, CN, CI, Br, F, CO-NOH-phenyl, OCH3, COCH3, or COOCH3; and in particular R2, R3, R4, R5 and R6 of B are H, OH, COOH, S03H, CH3, N02, CN, CI, Br, CO-NOH-phenyl, or OCH3.

B is preferably H or Ci-3-alkyl, said alkyi may contain hydroxy, ester or ether groups; preferably said alkyi may contain ester or ether groups; more preferably said alkyi may contain ether groups. In an embodiment, A and B independently of each other are: 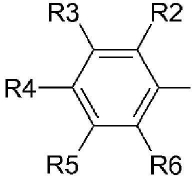 or B is H or Ci-3-alkyl, said alkyi may contain hydroxy, ester or ether groups (e.g. wherein the ether oxygen is directly attached to A-N(OH)C=0-, thus including N-hydroxy carbamic acid ester derivatives), and R2, R3, R4, R5 and R6 independently of each other are H, OH, NH2, COOH, S03H, Ci-3-alkyl, acyl, N02, CN, CI, Br, F, CF3, NOH-CO-phenyl, CO-NOH-phenyl, COR12, OR7, NR8R9, COOR10, or NOH-CO-R1 1 , wherein R7, R8 and R9 are C1-3-alkyl or acyl, and R10, R1 1 and R12 are C1-3-alkyl.

In another embodiment, A and B independently of each other are: 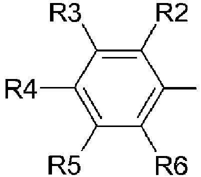 or B is H or Ci-3-alkyl, said alkyl may contain hydroxy or ether groups (e.g. wherein the ether oxygen is directly attached to A-N(OH)C=0-, thus including N-hydroxy carbamic acid ester derivatives), and R2, R3, R4, R5 and R6 independently of each other are H, OH, NH2, COOH, S03H, CH3, acyl, N02, CN, CI, Br, F, CF3, CO-NOH-phenyl, COCH3, OR7, NR8R9, or COOCH3, wherein R7, R8 and R9 are CH3 or COCH3.

In another embodiment, A and B independently of each other are: 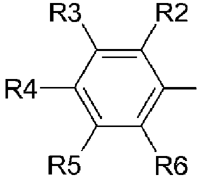 or B is H or Ci-3-alkyl, said alkyl may contain hydroxy or ether groups (e.g. wherein the ether oxygen is directly attached to A-N(OH)C=0-, thus including N-hydroxy carbamic acid ester derivatives), and R2, R3, R4, R5 and R6 independently of each other are H, OH, COOH, S03H, CH3, acyl, N02, CN, CI, Br, F, CO-NOH-phenyl, OCH3, COCH3, or COOCH3.

In another embodiment, A and B independently of each other are: 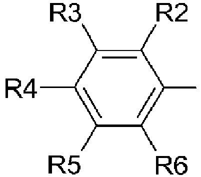 or B is Ci-3-alkyl, said alkyl may contain ether groups (e.g. wherein the ether oxygen is directly attached to A-N(OH)C=0-, thus including N-hydroxy carbamic acid ester derivatives), and R2, R3, R4, R5 and R6 independently of each other are H, OH, COOH, S03H, CH3, N02, CN, CI, Br, CO-NOH-phenyl, or OCH3.

The terms "Ci-n-alkyl" wherein n can be from 2 through 12, as used herein, represent a branched or straight alkyl group having from one to the specified number of carbon atoms. Typical Ci-6-alkyl groups include, but are not limited to, methyl, ethyl, n-propyl, iso-propyl, butyl, iso-butyl, sec-butyl, tert-butyl, pentyl, iso-pentyl, hexyl, iso-hexyl and the like.

The term "acyl" as used herein refers to a monovalent substituent comprising a Ci-6-alkyl group linked through a carbonyl group; such as e.g. acetyl, propionyl, butyryl, isobutyryl, pivaloyl, valeryl, and the like. In an embodiment, at least one of the substituents R2, R3, R4, R5 and R6 of A are H, preferably at least two of the substituents R2, R3, R4, R5 and R6 of A are H, more preferably at least three of the substituents R2, R3, R4, R5 and R6 of A are H, most preferably at least four of the substituents R2, R3, R4, R5 and R6 of A are H, in particular all of R2, R3, R4, R5 and R6 of A are H.

In another embodiment, at least one of the substituents R2, R3, R4, R5 and R6 of B are H, preferably at least two of the substituents R2, R3, R4, R5 and R6 of B are H, more preferably at least three of the substituents R2, R3, R4, R5 and R6 of B are H, most preferably at least four of the substituents R2, R3, R4, R5 and R6 of B are H, in particular all of R2, R3, R4, R5 and R6 of B are H.

In particular embodiments according to the invention, the mediator is selected from the group consisting of
4-nitrobenzoic acid-N-hydroxyanilide;
4-methoxybenzoic acid-N-hydroxyanilide;
N,N'-dihydroxy-N,N'-diphenylterephthalamide;
decanoic acid-N-hydroxyanilide;
N-hydroxy-4-cyanoacetanilide;
N-hydroxy-4-acetylacetanilide;
N-hydroxy-4-hydroxyacetanilide;
N-hydroxy-3-(N' -hydroxy acetamide)acetanilide;
4-cyanobenzoic acid-N-hydroxyanilide;
N-hydroxy-4-nitroacetanilide;
N-hydroxyacetanilide;
N-hydroxy-N-phenyl-carbamic acid isopropyl ester;
N-hydroxy-N-phenyl-carbamic acid methyl ester;
N-hydroxy-N-phenyl-carbamic acid phenyl ester;
N-hydroxy-N-phenyl-carbamic acid ethyl ester; and
N-hydroxy-N-(4-cyanophenyl)-carbamic acid methyl ester.

Another group of mediators is phenolic compounds (alkylsyringates) of the general formula IV: 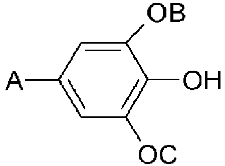 wherein the letter A in said formula denotes be a group such as -D, -CH=CH-D, -CH=CH-CH=CH-D, -CH=N-D, -N=N-D, or -N=CH-D, in which D is selected from the group consisting of - CO-E, -S02-E, -N-XY, and -NΓ-XYZ, in which E may be -H, -OH, -R, or -OR, and X and Y and Z may be identical or different and selected from -H and -R; R being a Ci-Ci6 alkyl, preferably a Ci-C8 alkyl, which alkyl may be saturated or unsaturated, branched or unbranched and optionally substituted with a carboxy, sulpho or amino group; and B and C may be the same or different and selected from CmH2m+i, where m = 1 , 2, 3, 4 or 5.

In the above mentioned general formula IV, A may be placed meta to the hydroxy group instead of being placed in the para-position as shown.

In particular embodiments of the invention, the mediator is selected from the group having the general formula V: 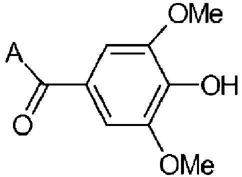 in which A is a group such as -H, -OH, -CH3, -OCH3, -0(CH2)nCH3, where n = 1 , 2, 3, 4, 5, 6, 7 or 8.

Yet another group of mediators are the compounds as described in general formula VI: 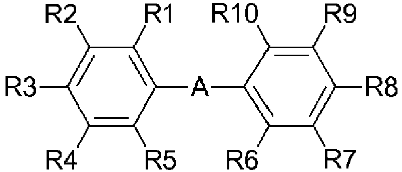
in which general formula A represents a single bond, or one of the following groups: (- CH2-), (-CH=CH-), (-NR1 1 -), (-CH=N-), (-N=N-), (-CH=N-N=CH-), or (>C=0);
and in which general formula the substituent groups R1 -R1 1 , which may be identical or different, independently represents any of the following radicals: hydrogen, halogen, hydroxy, formyl, acetyl, carboxy and esters and salts hereof, carbamoyl, sulfo and esters and salts hereof, sulfamoyl, methoxy, nitro, amino, phenyl, Ci-8-alkyl; which carbamoyl, sulfamoyl, phenyl, and amino groups may furthermore be unsubstituted or substituted once or twice with a substituent group R12; and which Ci-8-alkyl group may be saturated or unsaturated, branched or unbranched, and may furthermore be unsubstituted or substituted with one or more substituent groups R12; which substituent group R12 represents any of the following radicals: hydrogen, halogen, hydroxy, formyl, acetyl, carboxy and esters and salts hereof, carbamoyl, sulfo and esters and salts hereof, sulfamoyl, methoxy, nitro, amino, phenyl, or Ci-8-alkyl; which carbamoyl, sulfamoyl, and amino groups may furthermore be unsubstituted or substituted once or twice with hydroxy or methyl;
and in which general formula R5 and R6 may together form a group -B-, in which B represents a single bond, one of the following groups (-CH2-), (-CH=CH-), (-CH=N-); or B represents sulfur, or oxygen.

In particular embodiments of the invention, the mediator is selected from the group having the general formula VII: 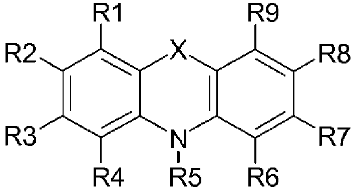
in which general formula X represents a single bond, oxygen, or sulphur;
and in which general formula the substituent groups R1 -R9, which may be identical or different, independently represents any of the following side groups: hydrogen, halogen, hydroxy, formyl, acetyl, carboxy and esters and salts hereof, carbamoyl, sulfo and esters and salts hereof, sulfamoyl, methoxy, nitro, amino, phenyl, Ci-8-alkyl; which carbamoyl, sulfamoyl, phenyl, and amino groups may furthermore be unsubstituted or substituted once or twice with a substituent group R10; and which Ci-8-alkyl group may be saturated or unsaturated, branched or unbranched, and may furthermore be unsubstituted or substituted with one or more substituent groups R10; which substituent group R10 represents any of the following radicals: hydrogen, halogen, hydroxy, formyl, acetyl, carboxy and esters and salts hereof, carbamoyl, sulfo and esters and salts hereof, sulfamoyl, methoxy, nitro, amino, phenyl, or Ci-8-alkyl; which carbamoyl, sulfamoyl, and amino groups may furthermore be unsubstituted or substituted once or twice with hydroxy or methyl. Another mediator according to the invention is 2,2',6,6'-tetramethyl- piperidine-/V-ox 1 (TEMPO):

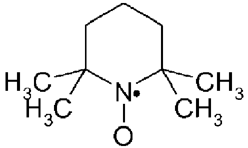

Preferred mediators are selected from the group consisting of 2,2'-azinobis-(3-ethylbenzthiazoline-6-sulfonate), 1-hydroxybenzotriazole, violuric acid, N-hydroxyacetanilide, methyl syringate, acetosyringone, syringaldezine, butyl syringate, pentyl syringate, hexyl syringate, heptyl syringate, vanillyl alcohol, synapic acid and acetovanillone, and mixtures thereof, particularly preferred are methyl syringate, acetosyringone, syringaldezine, butyl syringate, pentyl syringate, hexyl syringate, heptyl syringate, vanillyl alcohol, synapic acid, acetovanillone and mixtures thereof. These organic based mediators are preferably suited to be used with a laccase.

Transition metal coordination complexes can also be mediators. These compounds do not form radicals when oxidised by an oxidoreductase enzyme, and the electron exchange is centred on the metallic atom of the complex. This type of electron exchange involving only transition metal redox reactions allows the use of mediators with high stability in both oxidation states. This is a great advantage over the other type of mediators.

Several classes of peroxidase or oxidase mediators have been described, see US 5,700,769; and 5,965,510. Particular interest has been directed to the mediator phenothiazine-10-propionate. However, the described classes of mediators only enhance the peroxidase activity when hydrogen peroxide is added to the wash liquor. Other mediators are capable of enhancing the bleaching activity of the peroxidase enzyme with the addition of molecular oxygen, i.e. hydrogen peroxide does not need to be present for obtaining the desired enhancement of the oxidizing activity of peroxidases. Several classes of compounds can be envisaged which deliver the capability of enhancing the peroxidase activity, in the presence of only oxygen. Non-limiting examples include: the enhancer having the formula:

Z 1HN-NHZ2

wherein Z1, is any organic group e. g. (substituted) - (hetero) (polycyclic)-aromatic, substituted (cyclo)-alkyl containing hetero atoms, and Z2 is electron withdrawing group, selected from the group consisting of optionally substituted alkyl/(hetero)aryl- -sulfone, sulfoxide, - sulfonate, - carbonyl, -oxalyl, - amidoxalyl, 5 hydrazidoxalyl, -carboxyl and esters and salts thereof, amidyl, - hydrazidyl, nitrile.

A suitable mediator may have the formula:

ArHN-NHZ2

wherein Z2 is as defined before and Ar is an optionally substituted aromatic or heteroaromatic group e.g. phenyl, phenyl substituted with halogen(s), alkoxy, alkyl, (alkyl)amino substituents, pyridinyl, alkyl- pyridinyl, furanyl. In one aspect, enhancer compounds may have the generic structures: 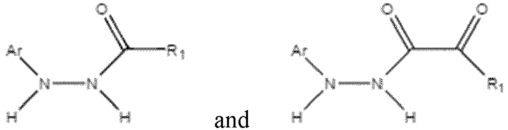
wherein the Ar group is as defined before and R1 is an optionally substituted alkyl, oxyalkyl, aryl, arylhydrazide, arylhydrazine or oxyaryl group, of interest are derivatives of 2'-phenylbenzohydrazide, having the following structure: 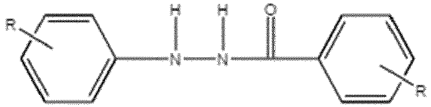
2-phenylhydrazide oxalate, having the following structure: 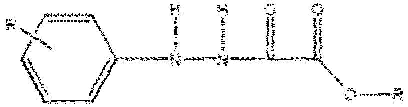
and oxalic acid bis(2-phenylhydrazide), having the following structure: 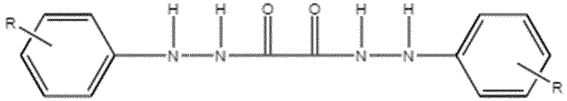
with R representing one or more substitutions independently selected from hydrogen, halogen(s), alkoxy, alkyl, (alkyl) amino, carbonate, carbonate ester, sulphonate, sulphonamide. Examples of such enhancers are: 2'-phenylbenzohydrazide; 2'-m-tolylbenzohydrazide; 2'-p-tolylbenzohydrazide; 2'-o-tolylbenzohydrazide; Ethyl [2-(m-tolyl)]hydrazide oxalate; Ethyl [2-(p-tolyl)]hydrazide oxalate; Ethyl [2-(o-tolyl)]hydrazide oxalate; Oxalic acid bis(2-phenylhydrazide); Oxalic acid bis(2-m-tolylhydrazide); and Oxalic acid bis(2-o- tolylhydrazide).

An especially preferred mediator for use herein is selected from the group consisting of phenoxazine-10-propionic acid, phenoxazine-10-hydroxyethyl, phenothiazine-10-ethyl-4-carboxy, phenothiazine-10-propionic acid, promazine hydrochloride, phenothiazine-10-ethylalcohol and a mixture thereof.

### Cleaning composition

The cleaning composition of the present invention is suitable for the cleaning of any type of surfaces when the cleaning involves the immersion of the surface in a wash liquor. The cleaning composition is suitable for use in hard surfaces and soft surfaces. It is particularly useful for use in laundry.

The cleaning composition of the present invention would comprise the customary ingredients for the cleaning process, such as surfactants and builders. The cleaning composition would preferably comprise components which can be combined under the term cleaning aids and which comprise different active ingredient groups such as foam regulators, bleaches, bleach activators and enzymes. Preferably the composition is free or bleach or comprises less than 5%, especially less than 1% by weight of the composition of bleach. The composition, especially when the composition is for use in laundry, can comprise cleaning auxiliaries including substances which are intended to prevent dyed textiles from causing a change in colour impression after the wash (dye transfer inhibitors). This colour change of washed, i.e. clean, textiles can be due to the fact that dye components are removed from the fabric ("fading") by the washing process, and on the other hand, dyestuffs released from differently coloured fabrics can be deposited on the textile ("discolouring"). Other cleaning auxiliaries include electrolytes, pH regulators and in the case of compositions for use in laundry, optical brightener, dye transfer inhibitors, fragrances, etc. In some embodiments the cleaning composition is free of bleach.

The composition preferably contains a surfactant or a plurality of surfactants, particularly anionic surfactants, nonionic surfactants and mixtures thereof, but it can also comprise cationic, zwitterionic and amphoteric surfactants.

Preferably the composition of the invention is a laundry cleaning composition. A laundry cleaning composition is any composition suitable to be used in a fabric laundering operation. The laundry cleaning composition may be in the form of a powder, a liquid or a mixture thereof.

The cleaning composition may comprise between 10% and 60%, preferably between 15% and 55%, more preferably between 20% and 50%, most preferably between 25% and 45% by weight of the composition of a surfactant system. Preferably, the surfactant system comprises a non-soap surfactant. Preferably, the surfactant system comprises an anionic surfactant and optionally a non-ionic surfactant. More preferably, the weight ratio of anionic surfactant to non-ionic surfactant is from 1:2 to 20:1, preferably from 1:1 to 15:1, more preferably from 1.5:1 to 10:1, most preferably from 5:1 to 10:1.

The non-soap anionic surfactant is preferably selected from sulphate or sulphonate anionic surfactants or mixtures thereof, preferably linear alkylbenzene sulphonate, alkyl sulphate, alkoxylated alkyl sulphate or a mixture thereof. Preferably, the alkoxylated alkyl sulphate is an ethoxylated alkyl sulphate preferably with an average degree of ethoxylation of between 0.5 and 4, preferably between 1 and 4, more preferably between 2 and 4, most preferably about 3.

Preferably, the weight ratio of linear alkylbenzene sulphonate to alkoxylated alkyl sulphate is between 15:1 and 1:3, preferably 10:1 and 1:2, more preferably 5:1 and 1:1, even more preferably 3:1 and 1:1, most preferably 2:1 and 1:1.

The non-ionic surfactant may be selected from a fatty alcohol alkoxylate, an oxosynthesised fatty alcohol alkoxylate, Guerbet alcohol alkoxylates, alkyl phenol alcohol alkoxylates, alkyl polyglucoside or a mixture thereof. Preferably, the non-ionic surfactant comprises a fatty alcohol ethoxylate non-ionic surfactant. Even more preferably the nonionic surfactant consists of a fatty alcohol ethoxylate surfactant.

Suitable fatty alcohol ethoxylate nonionic surfactants include the condensation products of aliphatic alcohols with from 1 to 25 moles of ethylene oxide. The alkyl chain of the aliphatic alcohol can either be straight or branched, guerbet, primary or secondary, and generally contains from 8 to 22 carbon atoms. The starting alcohol can be naturally derived, e.g. starting from natural oils, or synthetically derived, e.g. alcohols obtained from for example oxo-, modified oxo- or Fischer-Tropsch processes. Examples of oxo-process derived fatty alcohols include the Lial and Isalchem 5 fatty alcohols ex Sasol company and Lutensol fatty alcohols ex BASF company.

Examples of modified-oxo process derived fatty alcohols include the Neodol fatty alcohols ex Shell company. Fischer-Tropsch derived fatty alcohols include Safol fatty alcohols ex Sasol company. The alkoxylate chain of fatty alcohol ethoxylates is made up solely of ethoxylate groups. Preferably, the fatty alcohol ethoxylate non-ionic surfactant comprises on average 10 between 8 and 18, more preferably between 10 and 16 even more preferably between 12 and 15 carbon atoms in the alcohol carbon chain, and on average between 5 and 12, preferably between 6 and 10, more preferably between 7 and 8 ethoxy units in the ethoxylation chain. Preferably, the weight ratio of linear alkylbenzene sulphonate to non-ionic surfactant is between 2:1 to 20:1 preferably 2:1 and 10:1; more preferably 5:1 and 10:1.

Preferably, the weight ratio of alkoxylated alkyl sulphate to non-ionic surfactant is between 2:1 and 20:1 preferably between 2:1 and 10:1 more preferably between 2:1 and 5:1. Preferably, the weight ratio of linear alkylbenzene sulphonate to fatty alcohol ethoxylate non-ionic surfactant is between 2:1 to 20:1 preferably 2:1 and 10:1; more preferably 5:1 and 10:1. Preferably, the weight ratio of alkoxylated alkyl sulphate to fatty alcohol ethoxylate nonionic surfactant is between 2:1 and 20:1 preferably between 2:1 and 10:1 more preferably between 2:1 and 5:1.

The cleaning composition may comprise polymers, preferably selected from alkoxylated, preferably ethoxylated polyethyleneimine, alkoxylated polyalkyl phenol, a polyester terephthalate, hydroxyethylcellulose, preferably quaternized hydroxyethylcellulose, a carboxymethylcellulose or a mixture thereof.

The cleaning composition may comprise an adjunct material, wherein the adjunct material is preferably selected from cleaning polymers, soil suspension polymers, surface modifying polymers, builders, chelants, dispersants, enzymes, enzyme stabilizers, catalytic materials, bleach, bleach activators, polymeric dispersing agents, anti-redeposition agents, suds suppressors, aesthetic dyes, opacifiers, perfumes, perfume delivery systems, structurants, hydrotropes, rheology modifiers, processing aids, pigments and mixtures thereof. Having an adjunct material in the composition provides good overall cleaning, soil suspension and whiteness or colour brightness profile of the fabric to be treated.

### EXAMPLES

### Example 1

Immobilization of an oxidoreductase enzyme onto a solid substrate is done using EDC (N-(3-Dimethylaminopropyl)-N'ethylcarbodiimide) as described by Fischer MJ (Methods Mol Biol. 2010, 627:55-73). The activity of the immobilized oxidoreductase enzyme sample is confirmed by adding 600µl of syringaldazine onto 250ml of a liquid detergent solution. 0.06ppm of immobilized oxidoreductase enzyme is added to the solution and absorbance at 531nm is measured over a certain period of time. The starting colour of the solution is colourless/yellow with the end point being purple.

| Time (min) | Abs (nm) |
|---|---|
| 0 | 0.058 |
| 0.5 | 0.063 |
| 1 | 0.068 |
| 2 | 0.073 |
| 3 | 0.081 |
| 4 | 0.087 |
| 5 | 0.095 |
| 10 | 0.126 |
| 15 | 0.15 |
| 20 | 0.164 |
| 25 | 0.167 |

Cotton and polycotton fabrics including white and mixed coloured fabrics are washed together in a wash step comprising detergent composition 1. The wash water contains 13 litres water and from 30 to 60 g of detergent 1. The wash liquor also contains a sample of the immobilised enzyme described above.

The following are illustrative examples of cleaning compositions of the invention and are not intended to be limiting.

### Detergent Composition Examples 1-7: Heavy Duty Liquid laundry detergent compositions.

| **Ingredients** | **1** | **2** | **3** | **4** | **5** | **6** | **7** |
|---|---|---|---|---|---|---|---|
| | % weight | | | | | | |
| AE_{1.8}S | 6.77 | 5.16 | 1.36 | 1.30 | - | - | - |
| AE₃S | - | - | - | - | 0.45 | - | - |
| LAS | 0.86 | 2.06 | 2.72 | 0.68 | 0.95 | 1.56 | 3.55 |
| HSAS | 1.85 | 2.63 | 1.02 | - | - | - | - |
| AE9 | 6.32 | 9.85 | 10.20 | 7.92 | | | |
| AE8 | | | | | | | 35.45 |
| AE7 | | | | | 8.40 | 12.44 | |
| C₁₂₋₁₄ dimethyl Amine Oxide | 0.30 | 0.73 | 0.23 | 0.37 | - | - | - |
| C₁₂₋₁₈ Fatty Acid | 0.80 | 1.90 | 0.60 | 0.99 | 1.20 | - | 15.00 |
| Citric Acid | 2.50 | 3.96 | 1.88 | 1.98 | 0.90 | 2.50 | 0.60 |
| Optical Brightener 1 | 1.00 | 0.80 | 0.10 | 0.30 | 0.05 | 0.50 | 0.001 |
| Optical Brightener 3 | 0.001 | 0.05 | 0.01 | 0.20 | 0.50 | - | 1.00 |
| Sodium formate | 1.60 | 0.09 | 1.20 | 0.04 | 1.60 | 1.20 | 0.20 |
| DTI 1 | 0.32 | 0.05 | - | 0.60 | 0.10 | 0.60 | 0.01 |
| DTI 2 | 0.32 | 0.10 | 0.60 | 0.60 | 0.05 | 0.40 | 0.20 |
| Sodium hydroxide | 2.30 | 3.80 | 1.70 | 1.90 | 1.70 | 2.50 | 2.30 |
| Monoethanolamine | 1.40 | 1.49 | 1.00 | 0.70 | - | - | - |
| Diethylene glycol | 5.50 | - | 4.10 | - | - | - | - |
| Chelant 1 | 0.15 | 0.15 | 0.11 | 0.07 | 0.50 | 0.11 | 0.80 |
| 4-formyl-phenylboronic acid | - | - | - | - | 0.05 | 0.02 | 0.01 |
| Sodium tetraborate | 1.43 | 1.50 | 1.10 | 0.75 | - | 1.07 | - |
| Ethanol | 1.54 | 1.77 | 1.15 | 0.89 | - | 3.00 | 7.00 |
| Polymer 1 | 0.10 | - | - | - | - | - | 2.00 |
| Polymer 2 | 0.30 | 0.33 | 0.23 | 0.17 | - | - | - |
| Polymer 3 | - | - | - | - | - | - | 0.80 |
| Polymer 4 | 0.80 | 0.81 | 0.60 | 0.40 | 1.00 | 1.00 | - |
| 1,2-Propanediol | - | 6.60 | - | 3.30 | 0.50 | 2.00 | 8.00 |
| Structurant | 0.10 | - | - | - | - | - 0.10 | 0.10 |
| Perfume | 1.60 | 1.10 | 1.00 | 0.80 | 0.90 | 1.50 | 1.60 |
| Perfume encapsulate | 0.10 | 0.05 | 0.01 | 0.02 | 0.10 | 0.05 | 0.10 |
| Protease | 0.80 | 0.60 | 0.70 | 0.90 | 0.70 | 0.60 | 1.50 |
| Mannanase | 0.07 | 0.05 | 0.045 | 0.06 | 0.04 | 0.045 | 0.10 |
| Amylase 1 | 0.30 | - | 0.30 | 0.10 | - | 0.40 | 0.10 |
| Amylase 2 | - | 0.20 | 0.10 | 0.15 | 0.07 | - | 0.10 |
| Xyloglucannase | 0.20 | 0.10 | - | - | 0.05 | 0.05 | 0.20 |
| Lipase | 0.40 | 0.20 | 0.30 | 0.10 | 0.20 | - | - |
| Polishing enzyme | - | 0.04 | - | - | - | 0.004 | - |
| Nuclease | 0.05 | 0.03 | 0.01 | 0.03 | 0.03 | 0.003 | 0.003 |
| Dispersin B | - | - | - | 0.05 | 0.03 | 0.001 | 0.001 |
| Acid Violet 50 | 0.05 | - | - | - | - | - | 0.005 |
| Direct Violet 9 | - | - | - | - | - | 0.05 | - |
| Violet DD | - | 0.035 | 0.02 | 0.037 | 0.04 | - | - |
| Immobilized oxidoreductase enzyme | 0.5 | 0.03 | 0.005 | 0.05 | 0.5 | 0.03 | 0.005 |
| Oxidoreductase-mediator | 0.05 | - | - | - | 0.05 | - | - |
| Water, dyes & minors | Balance | | | | | | |
| pH | 8.2 | | | | | | |

Based on total cleaning and/or treatment composition weight. Enzyme levels are reported as raw material.

### Detergent Composition Examples 8 to 18: Unit Dose Compositions.

These examples provide various formulations for unit dose laundry detergents. Compositions 8 to 12 comprise a single unit dose compartment. The film used to encapsulate the compositions is a polyvinyl-alcohol-based film.

| **Ingredients** | **8** | **9** | **10** | **11** | **12** |
|---|---|---|---|---|---|
| | **% weight** | | | | |
| LAS | 19.09 | 16.76 | 8.59 | 6.56 | 3.44 |
| AE3S | 1.91 | 0.74 | 0.18 | 0.46 | 0.07 |
| AE7 | 14.00 | 17.50 | 26.33 | 28.08 | 31.59 |
| Citric Acid | 0.6 | 0.6 | 0.6 | 0.6 | 0.6 |
| C12-15 Fatty Acid | 14.8 | 14.8 | 14.8 | 14.8 | 14.8 |
| Polymer 3 | 4.0 | 4.0 | 4.0 | 4.0 | 4.0 |
| Chelant 2 | 1.2 | 1.2 | 1.2 | 1.2 | 1.2 |
| Optical Brightener 1 | 0.20 | 0.25 | 0.01 | 0.01 | 0.50 |
| Optical Brightener 2 | 0.20 | - | 0.25 | 0.03 | 0.01 |
| Optical Brightener 3 | 0.18 | 0.09 | 0.30 | 0.01 | - |
| DTI 1 | 0.10 | - | 0.20 | 0.01 | 0.05 |
| DTI 2 | - | 0.10 | 0.20 | 0.25 | 0.05 |
| Glycerol | 6.1 | 6.1 | 6.1 | 6.1 | 6.1 |
| Monoethanol amine | 8.0 | 8.0 | 8.0 | 8.0 | 8.0 |
| Tri-isopropanol amine | - | - | 2.0 | - | - |
| Tri-ethanol amine | - | 2.0 | - | - | - |
| Cumene sulfonate | - | - | - | - | 2.0 |
| Protease | 0.80 | 0.60 | 0.07 | 1.00 | 1.50 |
| Mannanase | 0.07 | 0.05 | 0.05 | 0.10 | 0.01 |
| Amylase 1 | 0.20 | 0.11 | 0.30 | 0.50 | 0.05 |
| Amylase 2 | 0.11 | 0.20 | 0.10 | - | 0.50 |
| Polishing enzyme | 0.005 | 0.05 | - | - | - |
| Nuclease | 0.005 | 0.05 | 0.005 | 0.010 | 0.005 |
| Dispersin B | 0.010 | 0.05 | 0.005 | 0.005 | - |
| Cyclohexyl dimethanol | - | - | - | 2.0 | - |
| Acid violet 50 | 0.03 | 0.02 | | | |
| Violet DD | | | 0.01 | 0.05 | 0.02 |
| Structurant | 0.14 | 0.14 | 0.14 | 0.14 | 0.14 |
| Perfume | 1.9 | 1.9 | 1.9 | 1.9 | 1.9 |
| Immobilized oxidoreductase enzyme | 0.5 | 0.03 | 0.005 | 0.05 | 0.5 |
| Oxidoreductase-mediator | 0.05 | - | - | - | 0.05 |
| Water and miscellaneous | To 100% | | | | |
| pH | 7.5-8.2 | | | | |

Based on total cleaning and/or treatment composition weight. Enzyme levels are reported as raw material.

In the following examples the unit dose has three compartments, but similar compositions can be made with two, four or five compartments. The film used to encapsulate the compartments is polyvinyl alcohol.

| **Base compositions Ingredients** | **13** | **14** | **15** | **16** |
|---|---|---|---|---|
| | **% weight** | | | |
| HLAS | 26.82 | 16.35 | 7.50 | 3.34 |
| AE7 | 17.88 | 16.35 | 22.50 | 30.06 |
| Citric Acid | 0.5 | 0.7 | 0.6 | 0.5 |
| C12-15 Fatty acid | 16.4 | 6.0 | 11.0 | 13.0 |
| Polymer 1 | 2.9 | 0.1 | - | - |
| Polymer 3 | 1.1 | 5.1 | 2.5 | 4.2 |
| Cationic cellulose polymer | - | - | 0.3 | 0.5 |
| Polymer 6 | - | 1.5 | 0.3 | 0.2 |
| Chelant 2 | 1.1 | 2.0 | 0.6 | 1.5 |
| Optical Brightener 1 | 0.20 | 0.25 | 0.01 | 0.005 |
| Optical Brightener 3 | 0.18 | 0.09 | 0.30 | 0.005 |
| DTI 1 | 0.1 | - | 0.2 | - |
| DTI 2 | - | 0.1 | 0.2 | - |
| Glycerol | 5.3 | 5.0 | 5.0 | 4.2 |
| Monoethanolamine | 10.0 | 8.1 | 8.4 | 7.6 |
| Polyethylene glycol | - | - | 2.5 | 3.0 |
| Potassium sulfite | 0.2 | 0.3 | 0.5 | 0.7 |
| Protease | 0.80 | 0.60 | 0.40 | 0.80 |
| Amylase 1 | 0.20 | 0.20 | 0.200 | 0.30 |
| Polishing enzyme | - | - | 0.005 | 0.005 |
| Nuclease | 0.05 | 0.010 | 0.005 | 0.005 |
| Dispersin B | - | 0.010 | 0.010 | 0.010 |
| MgCl₂ | 0.2 | 0.2 | 0.1 | 0.3 |
| Structurant | 0.2 | 0.1 | 0.2 | 0.2 |
| Acid Violet 50 | 0.04 | 0.03 | 0.05 | 0.03 |
| Perfume / encapsulates | 0.10 | 0.30 | 0.01 | 0.05 |
| Immobilized oxidoreductase enzyme | 0.5 | 0.03 | 0.05 | 0.5 |
| Oxidoreductase-mediator | 0.05 | - | - | 0.05 |
| Solvents and misc. | To 100% | | | |
| pH | 7.0-8.2 | | | |

| **Finishing compositions** | 17 | | | 18 | | |
|---|---|---|---|---|---|---|
| Compartment | A | B | C | A | B | C |
| Volume of each compartment | 40 ml | 5 ml | 5 ml | 40 ml | 5 ml | 5 ml |

| **Ingredients** | Active material in Wt.% | | | | | |
|---|---|---|---|---|---|---|
| Perfume | 1.6 | 1.6 | 1.6 | 1.6 | 1.6 | 1.6 |
| Violet DD | 0 | 0.006 | 0 | 0 | 0.004 | - |
| TiO2 | - | - | 0.1 | - | | 0.1 |
| Sodium Sulfite | 0.4 | 0.4 | 0.4 | 0.3 | 0.3 | 0.3 |
| Polymer 5 | - | | | 2 | - | - |
| Hydrogenated castor oil | 0.14 | 0.14 | 0.14 | 0.14 | 0.14 | 0.14 |
| Base Composition 13, 14, 15 or 16 | Add to 100% | | | | | |

Based on total cleaning and/or treatment composition weight, enzyme levels are reported as raw material.

### Detergent Composition Examples 19 to 24: Granular laundry detergent compositions for hand washing or washing machines, typically top-loading washing machines.

| **Ingredient** | **19** | **20** | **21** | **22** | **23** | **24** |
|---|---|---|---|---|---|---|
| | **% weight** | | | | | |
| LAS | 11.33 | 10.81 | 7.04 | 4.20 | 3.92 | 2.29 |
| Quaternary ammonium | 0.70 | 0.20 | 1.00 | 0.60 | - | - |
| AE3 S | 0.51 | 0.49 | 0.32 | - | 0.08 | 0.10 |
| AE7 | 8.36 | 11.50 | 12.54 | 11.20 | 16.00 | 21.51 |
| Sodium Tripolyphosphate | 5.0 | - | 4.0 | 9.0 | 2.0 | - |
| Zeolite A | - | 1.0 | - | 1.0 | 4.0 | 1.0 |
| Sodium silicate 1.6R | 7.0 | 5.0 | 2.0 | 3.0 | 3.0 | 5.0 |
| Sodium carbonate | 20.0 | 17.0 | 23.0 | 14.0 | 14.0 | 16.0 |
| Polyacrylate MW 4500 | 1.0 | 0.6 | 1.0 | 1.0 | 1.5 | 1.0 |
| Polymer 6 | 0.1 | 0.2 | - | - | 0.1 | - |
| Carboxymethyl cellulose | 1.0 | 0.3 | 1.0 | 1.0 | 1.0 | 1.0 |
| Acid Violet 50 | 0.05 | - | 0.02 | - | 0.04 | - |
| Violet DD | - | 0.03 | - | 0.03 | - | 0.03 |
| Protease 2 | 0.10 | 0.10 | 0.10 | 0.10 | - | 0.10 |
| Amylase | 0.03 | - | 0.03 | 0.03 | 0.03 | 0.03 |

| | | | | | | |
|---|---|---|---|---|---|---|
| Lipase | 0.03 | 0.07 | 0.30 | 0.10 | 0.07 | 0.40 |
| Polishing enzyme | 0.002 | - | 0.05 | - | 0.02 | - |
| Nuclease | 0.001 | 0.001 | 0.01 | 0.05 | 0.002 | 0.02 |
| Dispersin B | 0.001 | 0.001 | 0.05 | - | 0.001 | - |
| Optical Brightener 1 | 0.200 | 0.001 | 0.300 | 0.650 | 0.050 | 0.001 |
| Optical Brightener 2 | 0.060 | - | 0.650 | 0.180 | 0.200 | 0.060 |
| Optical Brightener 3 | 0.100 | 0.060 | 0.050 | - | 0.030 | 0.300 |
| Chelant 1 | 0.60 | 0.80 | 0.60 | 0.25 | 0.60 | 0.60 |
| DTI 1 | 0.32 | 0.15 | 0.15 | - | 0.10 | 0.10 |
| DTI 2 | 0.32 | 0.15 | 0.30 | 0.30 | 0.10 | 0.20 |
| Sodium Percarbonate | - | 5.2 | 0.1 | - | - | - |
| Sodium Perborate | 4.4 | - | 3.85 | 2.09 | 0.78 | 3.63 |
| Nonanoyloxybenzensulfonate | 1.9 | 0.0 | 1.66 | 0.0 | 0.33 | 0.75 |
| Tetraacetylehtylenediamine | 0.58 | 1.2 | 0.51 | 0.0 | 0.015 | 0.28 |
| Photobleach | 0.0030 | 0.0 | 0.0012 | 0.0030 | 0.0021 | - |
| S-ACMC | 0.1 | 0.0 | 0.0 | 0.0 | 0.06 | 0.0 |
| Immobilized oxidoreductase enzyme | 0.5 | 0.05 | 0.1 | 0.05 | 0.5 | 0.05 |
| Oxidoreductase-mediator | 0.05 | - | - | - | 0.05 | - |
| Sulfate/Moisture | Balance | | | | | |

### Detergent Composition Examples 25-30: Granular laundry detergent compositions typically for front-loading automatic washing machines.

| **Ingredient** | **25** | **26** | **27** | **28** | **29** | **30** |
|---|---|---|---|---|---|---|
| | **% weight** | | | | | |
| LAS | 6.08 | 5.05 | 4.27 | 3.24 | 2.30 | 1.09 |
| AE3 S | - | 0.90 | 0.21 | 0.18 | - | 0.06 |
| AS | 0.34 | - | - | - | - | - |
| AE7 | 4.28 | 5.95 | 6.72 | 7.98 | 9.20 | 10.35 |
| Quaternary ammonium | 0.5 | - | - | 0.3 | - | - |
| Crystalline layered silicate | 4.1 | - | 4.8 | - | - | - |
| Zeolite A | 5.0 | - | 2.0 | - | 2.0 | 2.0 |
| Citric acid | 3.0 | 4.0 | 3.0 | 4.0 | 2.5 | 3.0 |
| Sodium carbonate | 11.0 | 17.0 | 12.0 | 15.0 | 18.0 | 18.0 |
| Sodium silicate 2R | 0.08 | - | 0.11 | - | - | - |
| Optical Brightener 1 | - | 0.25 | 0.05 | 0.01 | 0.10 | 0.02 |
| Optical Brightener 2 | - | - | 0.25 | 0.20 | 0.01 | 0.08 |
| Optical Brightener 3 | - | 0.06 | 0.04 | 0.15 | - | 0.05 |
| DTI 1 | 0.08 | - | 0.04 | - | 0.10 | 0.01 |
| DTI 2 | 0.08 | - | 0.04 | 0.10 | 0.10 | 0.02 |
| Soil release agent | 0.75 | 0.72 | 0.71 | 0.72 | - | - |
| Acrylic /maleic acid copolymer | 1.1 | 3.7 | 1.0 | 3.7 | 2.6 | 3.8 |
| Carboxymethyl cellulose | 0.2 | 1.4 | 0.2 | 1.4 | 1.0 | 0.5 |
| Protease 3 | 0.20 | 0.20 | 0.30 | 0.15 | 0.12 | 0.13 |
| Amylase 3 | 0.20 | 0.15 | 0.20 | 0.30 | 0.15 | 0.15 |
| Lipase | 0.05 | 0.15 | 0.10 | - | - | - |
| Amylase 2 | 0.03 | 0.07 | - | - | 0.05 | 0.05 |
| Cellulase 2 | - | - | - | - | 0.10 | 0.10 |
| Polishing enzyme | 0.003 | 0.005 | 0.020 | - | - | - |
| Nuclease | 0.002 | 0.010 | 0.020 | 0.020 | 0.010 | 0.003 |
| Dispersin B | 0.002 | 0.010 | 0.020 | 0.020 | 0.010 | 0.002 |
| Tetraacetylehtylenediamine | 3.6 | 4.0 | 3.6 | 4.0 | 2.2 | 1.4 |
| Sodium percabonate | 13.0 | 13.2 | 13.0 | 13.2 | 16.0 | 14.0 |
| Chelant 3 | - | 0.2 | - | 0.2 | - | 0.2 |
| Chelant 2 | 0.2 | - | 0.2 | - | 0.2 | 0.2 |
| MgSO₄ | - | 0.42 | - | 0.42 | - | 0.4 |
| Perfume | 0.5 | 0.6 | 0.5 | 0.6 | 0.6 | 0.6 |
| Suds suppressor agglomerate | 0.05 | 0.10 | 0.05 | 0.10 | 0.06 | 0.05 |
| Soap | 0.45 | 0.45 | 0.45 | 0.45 | - | - |
| Acid Violet 50 | 0.04 | - | 0.05 | - | 0.04 | - |
| Violet DD | - | 0.04 | - | 0.05 | - | 0.04 |
| S-ACMC | 0.01 | 0.01 | - | 0.01 | - | - |
| Direct Violet 9 (active) | - | - | 0.0001 | 0.0001 | - | - |
| Immobilized oxidoreductase enzyme | 0.5 | 0.1 | 0.05 | 0.05 | 0.5 | 0.1 |
| Oxidoreductase-mediator | 0.2 | - | - | - | 0.2 | - |
| Sulfate/ Water & Miscellaneous | Balance | | | | | |

| | |
|---|---|
| AE1.8S | is C₁₂₋₁₅ alkyl ethoxy (1.8) sulfate |
| AE3 S | is C₁₂₋₁₅ alkyl ethoxy (3) sulfate |
| AE7 | is C₁₂₋₁₃ alcohol ethoxylate, with an average degree of ethoxylation of 7 |
| AE8 | is C₁₂₋₁₃ alcohol ethoxylate, with an average degree of ethoxylation of 8 |
| AE9 | is C₁₂₋₁₃ alcohol ethoxylate, with an average degree of ethoxylation of 9 |
| Amylase 1 | is Stainzyme^{®}, 15 mg active/g |
| Amylase 2 | is Natalase^{®}, 29 mg active/g |
| Amylase 3 | is Stainzyme Plus^{®}, 20 mg active/g, |
| AS | is C₁₂₋₁₄ alkylsulfate |
| Cellulase 2 | is Celluclean^{™} , 15.6 mg active/g |
| Xyloglucanase | is Whitezyme^{®}, 20mg active/g |
| Chelant 1 | is diethylene triamine pentaacetic acid |
| Chelant 2 | is 1-hydroxyethane 1,1-diphosphonic acid |
| Chelant 3 | is sodium salt of ethylenediamine-N,N'-disuccinic acid, (S,S) isomer (EDDS) |
| Dispersin B | is a glycoside hydrolase, reported as 1000mg active/g |
| DTI 1 | is poly(4-vinylpyridine-1-oxide) (such as Chromabond S-403E^{®}), |
| DTI 2 | is poly(1-vinylpyrrolidone-co-1-vinylimidazole) (such as Sokalan HP56^{®} ). |
| Dye control agent | Dye control agent in accordance with the invention, for example Suparex^{®} O.IN (M1), Nylofixan^{®} P (M2), Nylofixan^{®} PM (M3), or Nylofixan^{®} HF (M4) |
| HSAS | is mid-branched alkyl sulfate as disclosed in US 6,020,303 and US6,060,443 |

### Immobilized oxidoreductase

| | |
|---|---|
| enzyme | is immobilized oxidoreductase enzyme in accordance with the invention; for example Guardzyme^{®} 10.5mg/g. |
| LAS | is linear alkylbenzenesulfonate having an average aliphatic carbon chain length C₉-C₁₅ (HLAS is acid form). |
| Lipase | is Lipex^{®}, 18 mg active/g |
| Mannanase | is Mannaway^{®}, 25 mg active/g |
| Nuclease | is a Phosphodiesterase SEQ ID NO 1, reported as 1000mg active/g |
| Optical Brightener 1 | is disodium 4,4'-bis{[4-anilino-6-morpholino-s-triazin-2-yl]-amino}-2,2'-stilbenedisulfonate |
| Optical Brightener 2 | is disodium 4,4'-bis-(2-sulfostyryl)biphenyl (sodium salt) |
| Optical Brightener 3 | is Optiblanc SPL10^{®} from 3V Sigma |
| Oxidoreductase-mediator | is methyl syringate |
| Perfume encapsulate | is a core-shell melamine formaldehyde perfume microcapsules. |
| Photobleach | is a sulfonated zinc phthalocyanine |
| Polishing enzyme | is Para-nitrobenzyl esterase, reported as 1000mg active/g |
| Polymer 1 | is bis((C₂H₅O)(C₂H₄O)n)(CH₃)-N⁺-CₓH₂ₓ-N⁺-(CH₃)-bis((C₂H₅O)(C₂H₄O)n), wherein n = 20-30,x = 3 to 8 or sulphated or sulfonated variants thereof |
| Polymer 2 | is ethoxylated (EO₁₅) tetraethylene pentamine |
| Polymer 3 | is ethoxylated polyethylenimine |
| Polymer 4 | is ethoxylated hexamethylene diamine |
| Polymer 5 | is Acusol 305, provided by Rohm&Haas |
| Polymer 6 | is a polyethylene glycol polymer grafted with vinyl acetate side chains, provided by BASF. |
| Protease | is Purafect Prime^{®}, 40.6 mg active/g |
| Protease 2 | is Savinase^{®}, 32.89 mg active/g |
| Protease 3 | is Purafect^{®}, 84 mg active/g |
| Quaternary ammonium | is C₁₂₋₁₄ Dimethylhydroxyethyl ammonium chloride |
| S-ACMC | is Reactive Blue 19 Azo-CM-Cellulose provided by Megazyme |
| Soil release agent | is Repel-o-tex^{®} SF2 |
| Structurant | is Hydrogenated Castor Oil |
| Violet DD | is a thiophene azo dye provided by Milliken |

## Claims

1. A cleaning agent comprising a supporting substrate with an oxidoreductase enzyme immobilized thereon wherein the oxidoreductase enzyme is selected from oxidoreductase enzymes having an enzyme classification number selected from the group consisting of E.C. 1.1 - 1.10 and E.C. 1.12 - 1.99 and wherein the oxidoreductase enzyme is immobilized on the substrate by means of a covalent chemical bond.

2. A cleaning agent according to claim 1 where the oxidoreductase enzyme is a laccase.

3. A cleaning agent according to any of the preceding claims wherein the substrate is selected from the group consisting of fabrics, non-woven materials, plastics and inorganic particles.

4. A cleaning agent according to any of the preceding claims wherein the substrate is a tri-dimensional hollow body and preferably wherein the oxidoreductase enzyme is immobilised on the inside surface of the hollow body.

5. A cleaning agent according to any of the preceding claims wherein the substrate is an inorganic particle, preferably zeolite.

6. A cleaning kit comprising a cleaning composition and a cleaning agent according to any of the preceding claims.

7. A cleaning kit according to the preceding claim wherein the cleaning composition comprises a surfactant system preferably comprising an anionic surfactant and preferably comprising a non-ionic surfactant.

8. A cleaning kit according to claim 6 or claim 7 wherein the cleaning composition comprises an oxidoreductase-mediator, preferably selected from the group consisting of organic-based mediator, transition metal coordination complex mediator and mixtures thereof.

9. A cleaning kit according to claim 8 wherein the oxidoreductase mediator is selected from the group consisting of 2,2'-azinobis-(3-ethylbenzthiazoline-6-sulfonate), 1-hydroxybenzotriazole, violuric acid, N-hydroxyacetanilide, methyl syringate, acetosyringone, syringaldezine, butyl syringate, pentyl syringate, hexyl syringate, heptyl syringate, vanillyl alcohol, synapic acid, acetovanillone, phenoxazine-10-propionic acid, phenoxazine-10-hydroxyethyl, phenothiazine-10-ethyl-4-carboxy, phenothiazine-10-propionic acid, promazine hydrochloride, phenothiazine-10-ethylalcohol and mixtures thereof, preferably from the group consisting of methyl syringate, acetosyringone, syringaldezine, butyl syringate, pentyl syringate, hexyl syringate, heptyl syringate, vanillyl alcohol, synapic acid, acetovanillone and mixtures thereof.

10. A cleaning kit according to any of claims 6 to 9 further comprising a peroxidase, preferably wherein the peroxidase is immobilized on a substrate.

11. A method for cleaning surfaces with a wash liquor comprising the step of immersing the surface in a wash liquor and exposing the wash liquor to a cleaning agent according to any of claims 1 to 5 in a first wash step, optionally rinsing and drying the surface, and preferably subsequently separating the cleaning agent from the wash liquor and re-using the cleaning agent in a second wash step comprising immersing a second surface in a second wash liquor and exposing the second wash liquor to the cleaning agent from the first wash step.

12. Use of a cleaning agent according to any of claims 1 to 5 for the cleaning of a surface comprising immersing the surface into a wash liquor to reduce dye transfer in the wash liquor.

13. Use according to the claim 12 wherein the cleaning process is the laundry of fabrics, such that the surface comprises fabric and wherein the fabric comprises mixed colour fabrics.

## Patentansprüche

1. Reinigungsmittel, umfassend ein Trägersubstrat mit einem darauf immobilisierten Oxidoreductase-Enzym, wobei das Oxidoreductase-Enzym aus Oxidoreductase-Enzymen ausgewählt ist, die eine Enzymklassifizierungsnummer aufweisen, die ausgewählt ist aus der Gruppe bestehend aus E.C. 1,1 bis 1,10 und E.C. 1,12 bis 1,99 und wobei das Oxidoreductase-Enzym auf dem Substrat mittels einer kovalenten chemischen Bindung immobilisiert ist.

2. Reinigungsmittel nach Anspruch 1, wobei das Oxidoreductase-Enzym eine Laccase ist.

3. Reinigungsmittel nach einem der vorstehenden Ansprüche, wobei das Substrat ausgewählt ist aus der Gruppe bestehend aus Stoffen, Vliesmaterialien, Kunststoffen und anorganischen Teilchen.

4. Reinigungsmittel nach einem der vorstehenden Ansprüche, wobei das Substrat ein dreidimensionaler Hohlkörper ist und vorzugsweise wobei das Oxidoreductase-Enzym auf der Innenoberfläche des Hohlkörpers immobilisiert ist.

5. Reinigungsmittel nach einem der vorstehenden Ansprüche, wobei das Substrat ein anorganisches Teilchen, vorzugsweise Zeolith, ist.

6. Reinigungskit, umfassend eine Reinigungszusammensetzung und ein Reinigungsmittel nach einem der vorstehenden Ansprüche.

7. Reinigungskit nach dem vorstehenden Anspruch, wobei die Reinigungszusammensetzung ein Tensidsystem umfasst, vorzugsweise umfassend ein anionisches Tensid und vorzugsweise umfassend ein nichtionisches Tensid.

8. Reinigungskit nach Anspruch 6 oder 7, wobei die Reinigungszusammensetzung einen Oxidoreductase-Mediator umfasst, der vorzugsweise ausgewählt ist aus der Gruppe bestehend aus einem Mediator auf organischer Basis, einem Übergangsmetallkoordinationskomplexmediator und Mischungen davon.

9. Reinigungskit nach Anspruch 8, wobei der Oxidoreductase-Mediator ausgewählt ist aus der Gruppe bestehend aus 2,2'-Azinobis-(3-ethylbenzothiazolin-6-sulfonat), 1-Hydroxybenzotriazol, Violursäure, N-Hydroxyacetanilid, Methylsyringat, Acetosyringon, Syringaldezin, Butylsyringat, Pentylsyringat, Hexylsyringat, Heptylsyringate, Vanillylalkohol, Sinapinsäure, Acetovanillon, Phenoxazin-10-propionsäure, Phenoxazin-10-hydroxyethyl, Phenothiazin-10-ethyl-4-carboxy, Phenothiazin-10-propionsäure, Promazinhydrochlorid, Phenothiazin-10-ethylalkohol und Mischungen davon, vorzugsweise aus der Gruppe bestehend aus Methylsyringat, Acetosyringon, Syringaldezin, Butylsyringat, Pentylsyringat, Hexylsyringat, Heptylsyringat, Vanillylalkohol, Sinapinsäure, Acetovanillon und Mischungen davon.

10. Reinigungskit nach einem der Ansprüche 6 bis 9, ferner umfassend eine Peroxidase, vorzugsweise wobei die Peroxidase auf einem Substrat immobilisiert ist.

11. Verfahren zum Reinigen von Oberflächen mit einer Waschflotte, umfassend den Schritt eines Eintauchens der Oberfläche in eine Waschflotte und eines Aussetzens der Waschflotte einem Reinigungsmittel nach einem der Ansprüche 1 bis 5 in einem ersten Waschschritt, wahlweise ein Spülen und ein Trocknen der Oberfläche, und vorzugsweise ein anschließendes Trennen des Reinigungsmittels von der Waschflotte und ein erneutes Verwenden des Reinigungsmittels in einem zweiten Waschschritt, umfassend das Eintauchen einer zweiten Oberfläche in eine zweite Waschflotte und das Aussetzen der zweiten Waschflotte dem Reinigungsmittel aus dem ersten Waschschritt.

12. Verwendung eines Reinigungsmittels nach einem der Ansprüche 1 bis 5 für die Reinigung einer Oberfläche, umfassend das Eintauchen der Oberfläche in eine Waschflotte, um eine Farbstoffübertragung in der Waschflotte zu verringern.

13. Verwendung nach Anspruch 12, wobei der Reinigungsprozess die Wäsche von Stoffen ist, derart, dass die Oberfläche Stoffe umfasst und wobei der Stoff Stoffe aus gemischten Farben umfasst.

## Revendications

1. Agent de nettoyage comprenant un substrat de support avec une enzyme oxydoréductase immobilisée sur celui-ci dans lequel l'enzyme oxydoréductase est choisie parmi les enzymes oxydoréductase ayant un numéro de classification des enzymes choisi dans le groupe constitué par E.C. 1,1 - 1,10 et E.C. 1,12 - 1,99 et dans lequel l'enzyme oxydoréductase est immobilisée sur le substrat au moyen d'une liaison chimique covalente.

2. Agent de nettoyage selon la revendication 1, où l'enzyme oxydoréductase est une laccase.

3. Agent de nettoyage selon l'une quelconque des revendications précédentes, dans lequel le substrat est choisi dans le groupe constitué de tissus, matériaux non tissés, plastiques et particules inorganiques.

4. Agent de nettoyage selon l'une quelconque des revendications précédentes, dans lequel le substrat est un corps creux tridimensionnel et de préférence dans lequel l'enzyme oxydoréductase est immobilisée sur la surface intérieure du corps creux.

5. Agent de nettoyage selon l'une quelconque des revendications précédentes, dans lequel le substrat est une particule inorganique, de préférence une zéolite.

6. Kit de nettoyage comprenant une composition de nettoyage et un agent de nettoyage selon l'une quelconque des revendications précédentes.

7. Kit de nettoyage selon la revendication précédente, dans lequel la composition de nettoyage comprend un système d'agent tensioactif comprenant de préférence un agent tensioactif anionique et comprenant de préférence un agent tensioactif non ionique.

8. Kit de nettoyage selon la revendication 6 ou la revendication 7, dans lequel la composition de nettoyage comprend un médiateur d'oxydoréductase, de préférence choisi dans le groupe constitué de médiateur à base organique, médiateur complexe de coordination de métal de transition et leurs mélanges.

9. Kit de nettoyage selon la revendication 8, dans lequel le médiateur d'oxydoréductase est choisi dans le groupe constitué de 2,2'-azinobis-(3-éthylbenzthiazolin-6-sulfonate), 1-hydroxybenzotriazole, acide violurique, N-hydroxyacétanilide, syringate de méthyle, acétosyringone, syringaldezine, syringate de butyle, syringate de pentyle, syringate d'hexyle, syringate d'heptyle, alcool vanillique, acide synapique, acétovanillone, acide phénoxazin-10-propionique, acide phénoxazin-10-hydroxyéthyle, phénothiazin-10-éthyl-4-carboxy, acide phénothiazin-10-propionique, chlorhydrate de promazine, phénothiazine-10-éthylalcool et leurs mélanges, de préférence dans le groupe constitué de syringate de méthyle, acétosyringone, syringaldezine, syringate de butyle, syringate de pentyle, syringate d'hexyle, syringate d'heptyle, alcool vanillique, acide synapique, acétovanillone et leurs mélanges.

10. Kit de nettoyage selon l'une quelconque des revendications 6 à 9 comprenant en outre une peroxydase, de préférence dans lequel la peroxydase est immobilisée sur un substrat.

11. Procédé de nettoyage de surfaces avec une liqueur de lavage comprenant l'étape consistant à immerger la surface dans une liqueur de lavage et exposer la liqueur de lavage à un agent de nettoyage selon l'une quelconque des revendications 1 à 5 dans une première étape de lavage, éventuellement rincer et sécher la surface, et de préférence séparer ultérieurement l'agent de nettoyage de la liqueur de lavage et réutiliser l'agent de nettoyage dans une seconde étape de lavage comprenant l'immersion d'une seconde surface dans une seconde liqueur de lavage et l'exposition de la seconde liqueur de lavage à l'agent de nettoyage de la première étape de lavage.

12. Utilisation d'un agent de nettoyage selon l'une quelconque des revendications 1 à 5 destinée au nettoyage d'une surface comprenant l'immersion de la surface dans une liqueur de lavage pour réduire le transfert de colorant dans la liqueur de lavage.

13. Utilisation selon la revendication 12, dans laquelle le processus de nettoyage est la lessive de tissus, de telle sorte que la surface comprend un tissu et dans laquelle le tissu comprend des tissus de couleur mélangés.
